# DEMANDE DE BREVET EUROPEEN

(11) **EP 0 542 612 A1**
(43) Date de publication de la demande: **19.05.1993**
(21) Numéro de dépôt: 92403020.8
(22) Date de dépôt: 09.11.1992
(51) Int. Cl.: B01J 27/053, B01J 27/02

(54) **Utilisation d'un catalyseur en alkylation d'isobutane et/ou d'isopentane**

(30) Priorité: 14.11.1991 FR 9114154
(71) Demandeur: INSTITUT FRANCAIS DU PETROLE, F-92502 Rueil-Malmaison (FR)
(72) Inventeur: Joly, Jean-François, F-75003 Paris (FR); Perrin, Sylvie, F-92500 Rueil Malmaison (FR); Marcilly, Christian, F-78800 Houilles (FR)

(57) **Abrégé**

Catalyseur à base d'un support oxyde constitué d'au moins un oxyde sulfaté, et d'acide sulfurique, et son utilisation en alkylation catalytique d'isobutane et/ou d'isopentane en présence d'au moins une oléfine comportant de 3 à 6 atomes de carbone par molécule.

## Description

La présente invention concerne un catalyseur à base d'au moins un oxyde sulfaté et d'acide sulfurique et son utilisation en alkylation catalytique d'isobutane et/ou d'isopentane au moyen d'au moins une oléfine, qui permet d'obtenir au moins un produit par exemple dans le groupe constitué par les diméthylbutanes, les triméthylpentanes, les triméthylhexanes et les triméthylheptanes.

On sait que, pour alimenter les moteurs à combustion interne et à allumage commandé, et notamment les moteurs à taux de compression élevé, il est particulièrement intéressant de disposer de carburants à hauts indices d'octane, c'est-à-dire constitués essentiellement par des hydrocarbures paraffiniques fortement ramifiés. L'alkylation des isoparaffines (isobutane et isopentane) par les oléfines contenant de 3 à 6 atomes de carbone par molécule permet d'obtenir de tels produits. Cette réaction nécessite la mise en oeuvre de catalyseurs très acides, dans le but notamment de réduire les réactions parasites telles que les réactions d'abstraction d'hydrure de l'oléfine et de polymérisation qui fournissent des hydrocarbures peu ramifiés de faible indice d'octane et des hydrocarbures insaturés, les réactions de craquage et les réactions de dismutation.

Les procédés existant pour la production d'hydrocarbures par alkylation de l'isobutane par les oléfines utilisent soit l'acide sulfurique soit l'acide fluorhydrique comme catalyseur. Dans ces procédés le catalyseur acide constitue une phase liquide qui est mise en contact avec le mélange isobutane-oléfine liquide pour former une émulsion. Ces procédés sont coûteux et posent d'importants problèmes vis-à-vis de la sécurité des personnes et vis-à-vis de l'environnement. Afin de remédier à ces problèmes, des systèmes catalytiques différents des acides sulfurique et fluorhydrique en phase liquide ont été recherchés.

Pour catalyser les réactions d'alkylation des isoparaffines par les oléfines, on a déjà proposé de mettre au point des catalyseurs acides à partir de nombreux solides acides de natures différentes. Parmi les familles de catalyseurs acides on peut citer les tamis moléculaires (voir par exemple US-A-3.236.762, US-A-3.251.902, US-A-3.644.565, US-A-4.377.721, US-A-4.384.161 et US-A-4.300.015), les résines macroréticulaires éventuellement associées avec BF₃ (voir par exemple US-A-3.855.342, US-A-3.855.343, US-A-3.862.258 et US-A-3.879.489), les résines perfluorées de type NAFION (voir par exemple US-A-4.056.578 et US-A-4.038.213), les acides de Lewis et/ou de Bronsted déposés sur divers supports inorganiques (voir par exemple US-A-3.975.299, US-A-3.852.371 et US-A-3.979.476), les alumines chlorées (voir par exemple US-A-3.240.840, US-A-3.523.142, US-A-3.607.859, US-A-3.523.142, US-A-4.066.716, US-A-4.083.800 et US-A-4.066.716), les graphites intercalés par des acides de Lewis et/ou de Bronsted (voir par exemple US-A-4.083.885, US-A-4.116.880, US-A-4.128.596 et US-A-3.976.714) et les anions déposés sur supports oxydes tels que ZrO₂/SO₄ (voir par exemple J-01288329, J-01245953 et J-61242641 ). Ces solides conduisent à la production d'isoparaffines ramifiées mais souffrent de plusieurs défauts majeurs, parmi lesquels on peut citer l'utilisation de rapports molaires isobutane/oléfine souvent très élevés pour limiter l'importance des réactions secondaires et la faible stabilité dans le temps de l'activité catalytique (inhibition du catalyseur par dépôt d'oligomères insaturés) ; ces catalyseurs doivent alors être fréquemment régénérés. De plus, la faible acidité de certains solides acides, tels que les tamis moléculaires par exemple, impose l'utilisation de températures de réaction élevées ce qui est préjudiciable à l'obtention d'hydrocarbures d'indices d'octane élevés.

Dans la présente invention, on a découvert un nouveau catalyseur permettant d'obtenir des composés paraffiniques à hauts degrés de ramification et hauts indices d'octane par alkylation d'isoparaffine (isobutane et/ou isopentane) par au moins une oléfine comprenant de 3 à 6 atomes de carbone par molécule. Ce nouveau catalyseur est avantageusement mis en oeuvre dans un procédé où l'oléfine et/ou un mélange d'oléfines est introduit(e) dans le réacteur en phase liquide et en mélange avec l'isoparaffine et/ou un mélange d'isoparaffines. Le catalyseur est mis en oeuvre en lit fixe, lit mobile ou lit fluide, ou encore en suspension dans la phase liquide des réactifs soumise à une agitation efficace.

Le catalyseur de la présente invention renferme un support oxyde comprenant au moins un oxyde ayant subi un traitement préalable de sulfatation (nommé oxyde sulfaté dans la suite), et une phase acide comprenant de l'acide sulfurique, le support oxyde ayant été imprégné partiellement ou totalement par ladite phase acide. Les oxydes sulfatés sont connus comme étant des catalyseurs très acides et pouvant être utilisés dans la réaction d'isomérisation squelettale des paraffines courtes ; ces solides ont fait l'objet de nombreux brevets et publications, comme par exemple l'article de revue de K. ARATA : "solid superacids" dans Advances in Catalysis, volume **37**, pp. 165-211 (1991 ). Ces solides présentent une acidité trop forte pour la réaction d'alkylation des paraffines, il s'ensuit des réactions secondaires de craquage et de polymérisation de l'oléfine très importantes ce qui est préjudiciable à l'obtention d'alkylats de bonne qualité. Nous avons découvert que, d'une façon surprenante, il est possible d'obtenir un excellent catalyseur d'alkylation des paraffines en imprégnant par une phase acide comprenant de l'acide sulfurique, un support oxyde comprenant au moins un oxyde sulfaté tel que décrit par exemple dans l'article de revue cité ci-dessus. Parmi les support oxydes que l'on peut utiliser, on peut citer à titre d'exemple non limitatif : ZrO₂, TiO₂, SiO₂, SnO₂, Al₂O₃, Fe₂O₃, HfO₂ et toute combinaisons de deux au moins de ces composés (ZrO₂-TiO₂ par exemple). La présente invention s'applique à tout oxyde ayant été sulfaté à l'une quelconque des étapes de sa préparation et quelle que soit la teneur en soufre du composé final obtenu après traitement de calcination.

Un excellent catalyseur d'alkylation de paraffines est alors obtenu en imprégnant le support oxyde par une phase acide comprenant une solution concentrée d'acide sulfurique. La concentration de l'acide sulfurique est avantageusement comprise entre 5 et 100 % en poids, de préférence entre 50 et 100 % en poids et de manière encore plus préférée entre 88 et 100 % en poids. La surface spécifique du support oxyde que l'on utilise est comprise entre 0,01 et 1500 m²/g, de préférence entre 0,01 et 150 m²/g et de manière encore plus préférée entre 0,01 et 50 m²/g. Le volume poreux total du support oxyde est compris entre 0,005 et 1,5 cm³/g, de préférence entre 0,005 et 1 cm³/g. Lors de l'imprégnation dudit support oxyde, la phase acide comprenant la solution d'acide H₂SO₄ occupe une fraction du volume poreux total du support oxyde comprise entre 5 % et 100 %. Le catalyseur ainsi obtenu est caractérisé par une surface spécifique comprise entre 0,01 et 500 m²/g, de préférence entre 0,01 et 150 m²/g et de manière encore plus préférée entre 0,01 et 40 m²/g.

Il est possible d'ajouter dans la phase acide au moins un additif visant à améliorer les performances catalytiques. L'additif est choisi dans le groupe formé par H₃PO₄, BO₃H, BF₄H, FSO₃H, CF₃COOH, SbF₅ et CF₃SO₃H, de préférence choisi dans le groupe formé par FSO₃H, CF₃COOH, SbF₅ et CF₃SO₃H.

Le procédé de préparation du catalyseur selon l'invention comprend deux étapes. Dans une première étape le support oxyde est calciné à une température supérieure à 50 °C, de préférence supérieure à 80 °C et de manière encore plus préférée comprise entre 200 et 600°C, par exemple égale à environ 500 °C. La durée de cette étape de calcination est habituellement comprise entre 10 minutes et 50 heures. La calcination peut être effectuée en présence d'air ou de mélange air/azote, le débit étant compris entre 0,001 et 10 l/h/g. La seconde étape consiste en l'imprégnation du produit calciné par la phase acide. Pour réaliser cette étape on peut utiliser toutes les techniques bien connues de l'homme du métier. Le solide ainsi obtenu est alors conservé à l'abri de l'humidité, de préférence à une température inférieure à la température de cristallisation de la phase acide utilisée pour l'imprégnation. Ainsi conservé, le catalyseur selon la présente invention comprend donc au moins un oxyde sulfaté imprégné d'une phase acide solide comprenant de l'acide sulfurique.

Le catalyseur selon la présente invention est utilisé pur ou dilué avec divers matériaux présentant peu d'activité catalytique dans la réaction considérée comme par exemple la silice, l'alumine, la magnésie ou encore diverses argiles telles que par exemple la bentonite, la montmorillonite ou le kaolin.

Le mélange isoparaffine(s)-oléfine(s) est introduit dans le réacteur à une vitesse spatiale horaire, exprimée en poids d'oléfine introduite par unité de poids du catalyseur et par heure comprise entre 0,001 et 10 h⁻¹ et de préférence comprise entre 0,002 et 2 h⁻¹. Ledit mélange peut aussi être réalisé à l'intérieur du réacteur. Dans tous les cas le mélange ainsi constitué est dans le réacteur dans des conditions de pression et de température telles que le mélange d'hydrocarbures reste liquide sur le catalyseur et que les constituants du catalyseur restent à l'état solide.

La température de réaction peut être comprise entre -50 et 150 °C, mais nous avons découvert que, d'une façon surprenante, les performances catalytiques sont nettement améliorées lorsque la température de réaction est inférieure à la température de cristallisation de la phase acide utilisée pour l'imprégnation de la silice. La température de réaction doit alors être inférieure à +6 °C, de préférence à 0 °C et de manière encore plus préférée inférieure à -3 °C. La pression du réacteur est suffisante pour maintenir les hydrocarbures à l'état liquide dans le réacteur.

L'un des avantages du catalyseur selon l'invention tel que la phase acide est constituée principalement d'acide sulfurique est la possibilité d'alkyler l'isobutane et/ou l'isopentane à des températures inférieures à -10° C et pouvant atteindre -30° C. En effet, L. F. Albright et al. dans Ind. Eng. Chem. Res. **1988**, 27, pp. 381-397 indiquent très clairement l'intérêt qu'il y a à procéder à l'alkylation de l'isobutane en présence d'acide sulfurique à des températures inférieures à 0° C : baisse très importante des réactions secondaires, et donc de la consommation du catalyseur, augmentation de la qualité des hydrocarbures obtenus. Les résultats publiés ne font référence qu'à des essais réalisés à l'échelle du laboratoire. L'inconvénient lié à l'utilisation de telles températures est la nécessité d'une agitation extrêmement puissante étant donnée la très forte viscosité de l'acide sulfurique en solution à ces températures, voire même l'impossibilité d'agiter si la température est inférieure à la température de fusion de l'acide sulfurique. Le catalyseur selon la présente invention, lui, permet de procéder à l'alkylation de l'isobutane et/ou de l'isopentane à ces très basses températures sans nécessiter une augmentation de la puissance de l'agitation, la phase acide sulfurique étant contenue au sein de la porosité de la silice.

Afin de limiter les réactions secondaires, on peut utiliser un excès d'isoparaffines par rapport à l'oléfine. A titre d'exemple, dans le cas de l'alkylation de l'isobutane par un butène, l'isobutane peut être introduit pur dans la charge ou sous la forme d'un mélange de butanes contenant par exemple au moins 40 % d'isobutane. De plus, on peut introduire un butène pur ou encore un mélange de butènes isomères. Dans tous les cas, le rapport molaire isobutane/butènes dans la charge est compris entre 1 et 100, de préférence entre 3 et 50 et de manière souvent préférée entre 5 et 10.

Les produits de la réaction peuvent être contrôlés régulièrement par mesure de l'indice de brome, par exemple selon le projet de Norme Française Pr. M. 07.071 de mars 1969.

Lorsque la nature du catalyseur et les conditions de travail du catalyseur sont judicieusement choisies (en particulier la température), le catalyseur selon l'invention permet la production de produits d'alkylation des paraffines par les oléfines qui sont intéressants comme carburants pour les moteurs et constituants d'essence et qui comprennent par exemple au moins 60 % (en moles) de paraffines possédant 8 atomes de carbone par molécule et moins de 1 % (en moles) de composés non saturés, les paraffines comprenant 8 atomes de carbone par molécule comprenant 70 à 98 % (en moles) de triméthylpentanes.

Un autre avantage du catalyseur selon la présente invention est la possibilité d'alkyler, à basse température, l'isobutane avec des mélanges d'oléfines comportant de 3 à 6 atomes de carbone par molécule, où la proportion d'oléfines comportant 5 atomes de carbone par molécule et plus est très importante (au moins 10% en poids, de préférence au moins 40% en poids).

L'exemple suivant illustre l'invention sans toutefois en limiter la portée.

### Péparation du catalyseur

On active 16 g d'oxyde ZrO₂/SO₄ (3% en poids de soufre) de surface spécifique égale à 50 m²/g et de volume poreux total égal à 0,30 cm³/g, par calcination sous air pendant 4 heures à 500°C. Le solide ainsi activé est conservé sous argon. On procède alors à une imprégnation à sec de 14 g du solide par 7,7 g d'une solution d'acide sulfurique à 96 % en poids. Le solide ainsi obtenu contient 7,7 g d'acide sulfurique et 14 g d'oxyde ZrO₂/SO₄, et est conservé sous argon à -18 °C.

### Alkylation de l'isobutane par le butène-1

On introduit 21 g du catalyseur préparé selon la méthode décrite ci-dessus dans un réacteur en verre du type Fischer & Porter d'un volume de 360 ml préalablement purgé sous débit d'argon. Le réacteur contenant le catalyseur est alors fermé puis mis sous vide primaire, puis il est refroidi à la température de -20 °C.

57 cm³ d'isobutane sont alors ajoutés dans le réacteur contenant le catalyseur sous agitation, ledit réacteur étant immergé dans un bain froid à -20 °C. On laisse sous agitation le système catalyseur + isobutane pendant une durée de 30 minutes afin d'homogénéiser la température.

On ajoute régulièrement 1,70 cm³ de butène-1 par heure pendant 6 heures, la température du réacteur étant maintenue à -7 °C pendant toute la durée de l'injection.

Après réaction, la phase hydrocarbure est soutirée du réacteur, l'isobutane est évaporé lentement. On recueille l'alkylat qui est analysé par chromatographie en phase vapeur ; sa composition pondérale est donnée ci-dessous. La conversion de l'oléfine est de 100 %.

| | |
|---|---|
| iC₅ | 1,26 |
| C₆ | 3,16 |
| C₇ | 2,62 |
| C₈ | 83,00 |
| C₉ | 1,70 |
| C₁₀ | 1,03 |
| C^{10⁺} | 7,23 |

La fraction C₈ contient 89,7% poids de triméthylpentanes, la fraction C₉ contient 92,2% de triméthylhexanes.

## Revendications

1. Catalyseur comprenant un support oxyde comprenant au moins un oxyde sulfaté et une phase acide à l'état solide comprenant de l'acide sulfurique de concentration comprise entre 5 et 100 % en poids, le support oxyde ayant été imprégné par ladite phase acide et ayant une surface spécifique comprise entre 0,01 et 1500 m²/g et un volume poreux total compris entre 0,005 et 1,5 cm³/g.

2. Catalyseur selon la revendication 1 tel que ladite phase acide comprend en outre au moins un additif..

3. Catalyseur selon l'une des revendications 1 ou. 2 tel que l'additif est choisi dans le groupe formé par FSO₃H, CF₃SO₃H, SbF₅ et CF₃COOH.

4. Procédé de préparation d'un catalyseur selon l'une des revendications 1 à 3 tel que le support oxyde est calciné puis imprégné par ladite phase acide.

5. Utilisation du catalyseur, selon l'une des revendications 1 à 3 ou préparé selon la revendication 4, dans un procédé d'alkylation catalytique d'au moins un élément choisi dans le groupe formé par l'isobutane et l'isopentane par au moins un élément choisi dans le groupe formé par les oléfines comprenant de 3 à 6 atomes de carbone par molécule.

6. Utilisation selon la revendication 5 dans laquelle la température de la réaction est inférieure à 6°C.

7. Utilisation selon l'une des revendications 5 ou 6 dans laquelle la température est inférieure à 0° C.
